(19) Europäisches Patentamt
European Patent Office
Office européen des brevets

(11) **EP 2 442 866 B1**

(12) **EUROPEAN PATENT SPECIFICATION**

(45) Date of publication and mention
of the grant of the patent:
**16.10.2013  Bulletin 2013/42**

(51) Int Cl.:
***A61N 1/378*** *(2006.01)*    ***H01G 9/052*** *(2006.01)*

(21) Application number: **10727250.2**

(22) Date of filing: **07.06.2010**

(86) International application number:
**PCT/US2010/037574**

(87) International publication number:
**WO 2010/147778 (23.12.2010 Gazette 2010/51)**

(54) **VOLUMETRIC ENERGY DENSITY ELECTRODES**

ELEKTRODEN MIT VOLUMETRISCHER ENERGIEDICHTE

ELECTRODES À DENSITÉ D'ÉNERGIE VOLUMÉTRIQUE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **16.06.2009  US 485215**

(43) Date of publication of application:
**25.04.2012  Bulletin 2012/17**

(73) Proprietor: **Medtronic, Inc**
**Minneapolis, Minnesota 55432 (US)**

(72) Inventors:
• **HINTZ, Michael B.**
**Mahtomedi, Minnesota 55115 (US)**

• **YOUNG, Paul B.**
**New Richmond, Wisconsin 54017 (US)**
• **HOSSICK-SCHOTT, Joachim**
**Minneapolis, Minnesota 55419 (US)**

(74) Representative: **Hughes, Andrea Michelle**
**Dehns**
**St Bride's House**
**10 Salisbury Square**
**London EC4Y 8JD (GB)**

(56) References cited:
**WO-A1-02/45109    US-A- 3 818 581**
**US-A- 4 537 641    US-A1- 2008 145 262**
**US-B1- 6 593 532**

**Description**

FIELD

The present teachings relate to high volumetric energy density electrolytic capacitors. More specifically the present teachings relate to electrolytic capacitor anodes having operating voltages greater than 90% of the forming voltage used to form their dielectric oxide.

INTRODUCTION

Efforts have been made to create capacitors which are smaller and useful in space critical applications, such as aerospace, military, and medical applications. In the context of medical devices, capacitors are typically charged and discharged rapidly for delivery of low voltage or high voltage stimuli. Upon or during detection of a potentially lethal arrhythmia, suitable electrical transformer circuitry charges one or more high voltage capacitors using a low voltage battery as a charge source. Then, at an appropriate time the energy stored in the capacitor discharges through a pair of electrodes disposed in or near a patient's heart. The discharged energy is used to terminate the arrhythmia and restore organized cardiac activity.

To further enhance the capacitor and minimize the size of the capacitor, the volumetric energy density (VED) or the quantity of electrical energy that can be stored per unit volume, can be optimized. However, known techniques have not addressed the relationship between the volumetric energy density and the working voltage ($V_w$) to forming voltage ($V_f$) ratios for space critical applications. The present teachings optimize the volumetric energy density in space critical applications and optimize operations of the capacitors at high working voltage to forming voltage ratios.

WO 02/45109 discloses a method of producing pellets for use in electrolytic capacitors; US 3,818,581 describes a process for producing a capacitor electrode.

SUMMARY

This section provides a general summary of the disclosure, and is not a comprehensive disclosure of its full scope or all of its features.

The present teachings provide methods of preparing an anode for use in a high volumetric energy density electrolytic capacitor. A valve metal powder compact having a  lead wire embedded therein is de-oxidized and sintered in the presence of a reactive metal having a stronger affinity for oxygen than the valve metal powder to form the anode. The reactive metal and at least one other contaminant are leached from the sintered powder structure. A residue within the anode from the reactive metal is then redistributed by thermal processing. A dielectric oxide is anodically formed on a surface of the anode. The capacitor contains the anode and has an operating voltage greater than 90% of the specified forming voltage.

The present teachings provide methods of administering a therapy to a patient. An anode is provided having an operating voltage greater than 90% of the forming voltage and is implanted into the patient. A therapy is provided with the implant and the anode facilitates an operating voltage greater than 90% of the forming voltage.

The present teachings further provide a valve metal capacitor anode including a valve metal lead wire embedded within a metal powder compact. The valve metal lead wire and metal powder compact are bonded together during a de-oxidation sintering process in the presence of a reactive metal vapor, subsequently leached to remove a residue from the reactive metal vapor, and thermally processed to redistribute remaining residue from the reactive metal vapor.

Further areas of applicability will become apparent from the description provided herein. The description and specific examples in this summary are intended for purposes of illustration only and are not intended to limit the scope of the present disclosure. The invention is set out in the appended claims.

DRAWINGS

The drawings described herein are for illustrative purposes only of selected embodiments and not all possible implementations, and are not intended to limit the scope of the present disclosure.

Corresponding reference numerals indicate corresponding parts throughout the several views of the drawings.

Figure 1 depicts an implantable medical device in a patient according to various embodiments;
Figure 2 depicts a capacitor according to various exemplary embodiments;
Figure 3 depicts an anode according to various exemplary embodiments;
Figure 4 depicts a de-oxidation sintering process of an anode according to various embodiments;
Figure 5 depicts a schematic comparison of specific charge decrease with formation potential for high and low specific charge powders for anodes according to various embodiments;

Figure 6 depicts an initial constant current charge behavior for anodes according to various embodiments;

Figure 7 depicts a constant current charging behavior for thermal treatment histories of anodes according to various embodiments; and

Figure 8 depicts secondary ion mass spectrometry (SIMS) magnesium depth profiles in tantalum lead wires of anodes according to various embodiments.

DETAILED DESCRIPTION

Exemplary embodiments will now be described more fully with reference to the accompanying drawings.

The embodiments or examples of the present disclosure which do not fall within the scope of the appended claims are provided for illustrative purposes only and do not form part of the present invention.

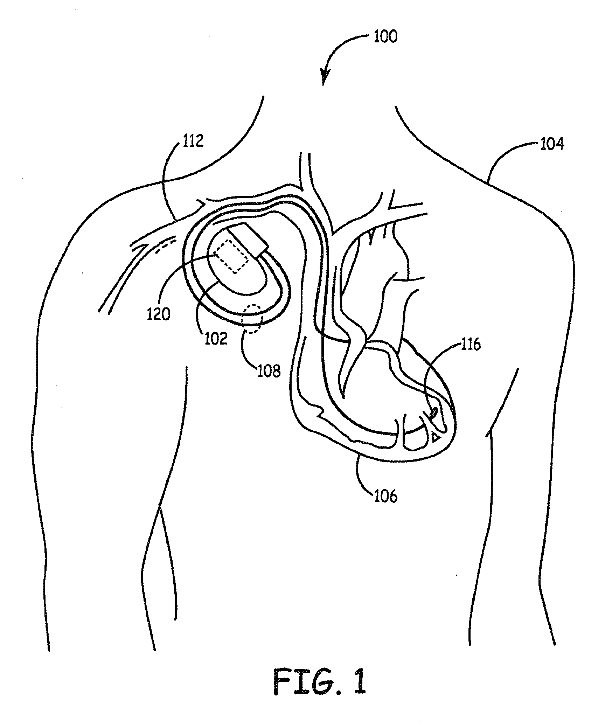

In various exemplary embodiments, and with reference to Figures 1-8, the present teachings provide methods and devices which optimize and increase the volumetric energy density (VED) of a capacitor 20. In particular, Applicants have found that prior treatments including a combination of de-oxidation sintering and an acid leaching, such as those described in U.S. Patent No. 4,537,641, result in increased specific charge upon dielectric oxide formation. It was found that anodes produced using those described process steps exhibited significant gas evolution and high leakage currents upon charging in a suitable working electrolyte to voltages greater than about 80% of the formation voltage. Applicants then discovered that a separate vacuum thermal treatment step following the leaching process can result in significant reduction in gas evolution and leakage currents in finished anodes produced by subsequent dielectric oxide formation. This process provides the high operating voltage to working voltage ratios detailed and claimed herein.

The energy, U, stored in the capacitor 20 is given by:

$$U = \frac{1}{2}CV^2 \qquad [1]$$

Where C is the capacitance in Farads and V can represent the $V_w$ or operating or working electrical potential to which the capacitor 20 is charged in volts. In order to maximize VED, it is therefore necessary to maximize both C and $V_w$ per unit volume. For the purposes of illustration, one can consider the capacitance of a parallel plate capacitor, which is given by:

$$C = \varepsilon\varepsilon_0 \frac{A}{d} \qquad [2]$$

Where $\varepsilon_0$ is the permittivity of free space, A is the plate surface area, $\varepsilon$ is the dielectric constant for the dielectric material between the plates, and d is the separation between the plates. $\varepsilon$ for an electrolytic capacitor is substantially fixed by the choice of the metal-metal oxide system. Maximizing capacitance per unit volume comprises maximizing A and minimizing d.

The effective separation between the plates (not shown) of an electrolytic capacitor is substantially equal to the dielectric film thickness which is linearly proportional to the formation voltage or forming voltage $V_f$. Consequently, it immediately follows that for a given $V_w$ dictated by the application, it is advantageous to operate the capacitor at the highest $V_w / V_f$ ratios consistent with other performance constraints such as leakage current and device lifetime.

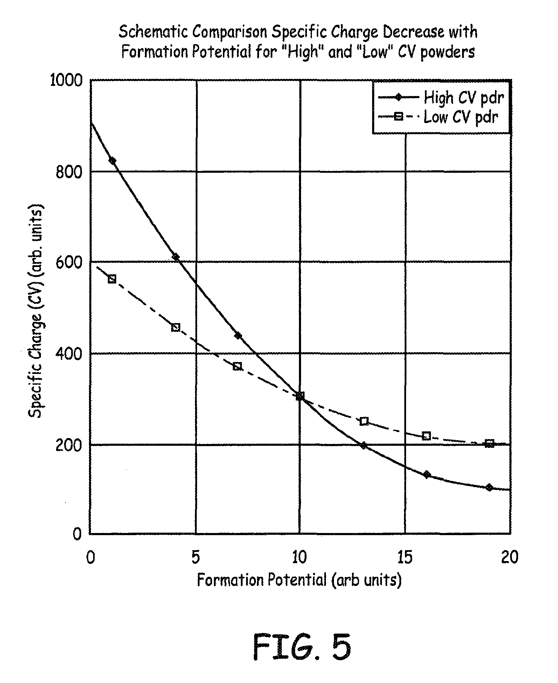

An additional or alternate approach for increasing VED is to increase C per unit volume by increasing the effective area (A) per unit volume. A variety of capacitor grade metal powders, described here as tantalum powders, are rated at an expected CV per unit weight or per unit volume, as used in the art, or specific charge in the finished part. The actual CV obtained for the finished device is a function of $V_f$. CV decreases much more rapidly with $V_f$ for high CV or finer powders than for low CV or coarser powders, as shown in Figure 5. Accordingly, the actual CV realized in the final device only remains large at low values of $V_f$.

The formation voltage $V_f$ needed for the particular application is an important input parameter for selection of powder size and optimization of area (A) per unit volume. During formation, oxygen supplied by the electrolyte combines with metal from the porous sintered metal powder compact, such as tantalum to produce the amorphous tantalum pentoxide ($Ta_2O_5$) dielectric film, as a non-limiting example. Consequently, formation of the dielectric consumes the underlying tantalum metal, thereby reducing the cross section of metallic material in the underlying porous sintered metal powder structure. It follows that more of the underlying porous sintered metal powder structure is consumed as formation voltage is increased. Upon completion of formation, the resulting dielectric film only contributes to useable capacitance if a contiguous conductive path exists between the remaining metal electrode upon which the dielectric oxide was formed and the anode connection terminal or lead wire 32. Consequently, the desired minimum structural feature size in the porous sintered metal powder compact increases as formation voltage increases. Powder particles and, particularly, the inter-particle bonds formed during sintering that are too small for the forming voltage employed are completely consumed and make a negligible contribution to capacitance in the final device. The formation voltage needed for the application therefore impacts selection of an appropriate powder, development of an optimal sintering process, and processing steps.

In various exemplary embodiments, and with reference to Figure 1, the present teachings employ the above-described concepts to optimize the VED in an implantable medical device (IMD) system 100 which includes an implantable medical device 102. The implantable medical device 102 can be a defibrillator and may be implanted within a patient 104 for providing a therapeutic electric shock to the patient's heart 106. It will be appreciated however, that the implantable medical device 102 can be in the form of various other medical devices including, but not necessarily limited to, a pacemaker, a cardioverter, neural stimulator, drug administering device, etc. without departing from the scope of the present teachings.

The implantable medical device 102 can be housed within a hermetically sealed, biologically inert outer container or housing, which may itself be conductive and also serve as an electrode, if appropriate for the IMD application. One or more leads, collectively identified with reference numeral 108 in Figure 1, are electrically coupled to the implantable device 102. The leads 108 can extend into the patient's heart 106 via a vein 112. The leads 108 can have one or more exposed conductive electrodes 116 for sensing cardiac activity and/or providing a stimulating voltage to the heart 106.

In accordance with the illustrated exemplary embodiment, the implantable medical device 102 comprises a capacitor bank 120, which includes one or more capacitors (not shown) disposed therein that store energy provided by a battery (not shown) within the implantable medical device 102. In one exemplary embodiment of the present teachings, the capacitor bank 120 stores the energy from the battery to deliver a therapeutic electric shock via the leads 108 to defibrillate the patient's heart 106. That is, when the implantable medical device 102 determines that the cardiac therapy/ stimulating electric shock is needed to produce a normal sinus rhythm of the heart 106, the capacitors in the capacitor bank 120 are charged to a pre-determined charge level by the battery. When the implantable medical device 102 determines that a therapeutic electric shock is needed, the charge stored in the capacitors is released by discharging the capacitors of the capacitor bank 120 through the patient's heart tissue via the leads 108.

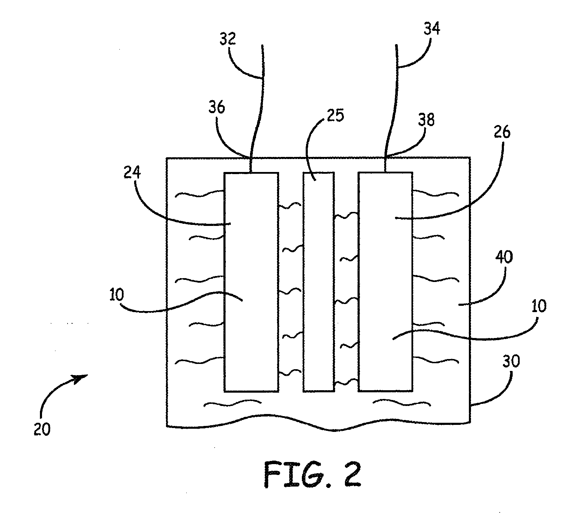

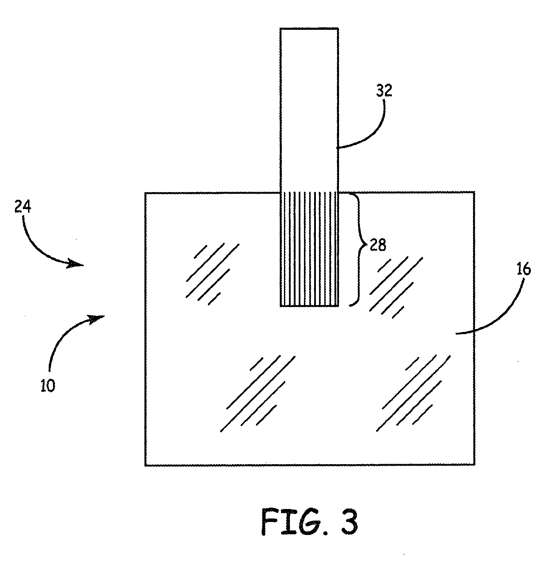

Referring to Figures 2 and 3, in various exemplary embodiments, the present teachings provide methods of forming an electrode 10. With specific reference to Figure 2, the electrodes 10 are disclosed in connection with the capacitor assembly 20. The capacitor assembly 20 can be assembled from an anode 24, a cathode 26, and a separator 25 between the anode 24 and the cathode 26 and fitted into the capacitor case 30 with appropriate electrical connectors 32, 34 to the anode 24 and cathode 26. In other exemplary embodiments, a capacitor 20 can be assembled from the anode 24, a cathode 26, and a separator 25 between the anode 24 and the cathode 26, and a plurality of the layers 24, 26 are stacked into a capacitor sub-assembly, electrically interconnected and fitted into a capacitor case 30 with appropriate electrical connectors 32, 34 to the anode 24 and cathode 26. The capacitor case 30 can be filled with a fluid electrolyte 40 which provides a current path between the anode 24 and the cathode 26. The electrodes 10 of the present teachings are able to carry a high voltage of from about 150 to about 375 volts.

The anode 24 can be electrically coupled to an anode lead wire 32 or pin. The anode lead wire 32 passes through the inner and outer surfaces of the capacitor case 30 via a feed-through 36. It should be understood that the term "anode" as used herein may refer to parts in process that will eventually become capacitor anodes following completion of all process steps. Consequently, a pressed powder body 16 comprising an embedded pin 32, the part that results when the pressed powder body 16 and pin 32 are processed through de-oxidation sintering, as well as the finished part after anodic oxide formation, etc. may all be referred to as anodes, as shown in Figure 3.

The various parts of the anode 24 can be made of any suitable metal including, but not limited to, aluminum, tantalum, niobium, titanium, zirconium, etc., all of which form adherent, electrically insulating metal-oxide films upon anodic polarization in electrically conductive solutions. Combinations of the above-identified materials and others are also within the scope of the present teachings.

The anode 24, in part, can form a porous metal sponge or body 16 by sintering tantalum metal powder, for example, which has been pressed into a desired shape. The porous and sponge-like nature of the anode 24 yields a high ratio of anode surface area to anode volume which is desirable for high volumetric energy density. However, the large surface area facilitates an adsorption of potentially detrimental contaminants. Applicants have discovered that contamination by magnesium during the processing described herein detrimentally limits the working voltage of anode 24. Particularly, this contamination can lead to a working voltage which is significantly less than the forming voltage of the anode 24.

In various exemplary embodiments, and with further reference to Figure 3, the lead wire 32 can be pre-treated to improve subsequent bonding to the anode body 16. A small volume of metal powder can be applied to the lead wire 32 at a contact region 28 and subsequently vacuum sintered onto a portion of the lead wire 32 to form a "prepared" lead wire or pin 32. Pre-treatment is described in U.S. Patent Application Serial No. 12/429,749, filed on April 24, 2009, and commonly owned with the present application, the disclosure of which is incorporated herein by reference.

To form anodes 24 of the present teachings, de-oxidation sintering can be employed. De-oxidation sintering differs from conventional vacuum sintering in that deoxidation sintering can be conducted at a lower temperature and in a selected gaseous environment where the sintering kinetics can be appreciably different than those exhibited in conventional vacuum sintering. Applicants have found that the de-oxidation sintering process facilitates forming smaller capacitors for space-critical applications which are capable of handling high voltage and is therefore beneficial.

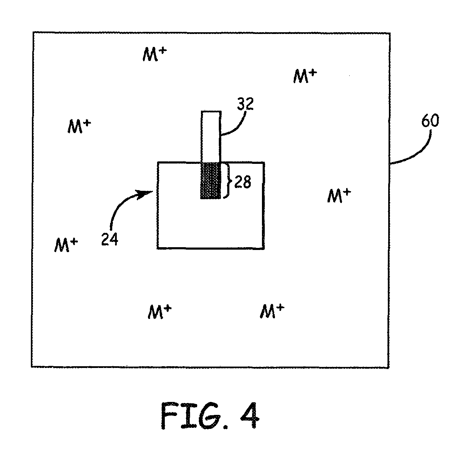

De-oxidation and de-oxidation sintering comprise applying heat and pressure in a metal vapor (M+) atmosphere. For example, the de-oxidation sintering of tantalum capacitor anodes can be conducted at a temperature of about 1150 degrees C. This temperature is significantly lower than the 1350 to 1600 degrees C temperature used in the traditional vacuum sintering process. As shown in Figure 4, the de-oxidation sintering can be conducted in a vessel 60, which in various embodiments can be a substantially hermetic vessel, in the presence of a metal vapor, indicated by the symbol $M^+$. It is understood that the vessel 60 can also represent an oven or furnace according to various embodiments of the present teachings. The metal which forms the metal vapor atmosphere generally has a higher affinity for oxygen than the particulate metal used for the anode 24. For example, if the metal comprises tantalum, the metal vapor could comprise a metal having a higher affinity for oxygen. In various exemplary embodiments, the metal vapor atmosphere is selected from the group of magnesium, calcium, and sodium, and combinations thereof.

In exemplary embodiments, the presence of the reactive metal vapor M+ may cause formation of an oxide layer or a contaminant on the electrode 10 surface. For example, a magnesium oxide layer can form where a magnesium vapor can be used in the de-oxidation sintering process. Further, the magnesium vapor and the materials used for the anode may form residue on the anode 24. The residue or contaminant can include magnesium, oxides, and/or reactive intermediates formed during the deoxidation and sintering process, as non-limiting examples.

To remove the residual magnesium, oxide, or reaction products, the electrode 10 can be subjected to a leaching treatment or first removal of the residue. In various exemplary embodiments, the leaching treatment is an acid treatment, such as a bath, dip, or spray that removes the oxides without damaging the underlying substrate. Suitable acids for the treatment include weak solutions of an inorganic acid, such as hydrochloric acid or sulfuric acid, as non-limiting examples. Other solutions may also be employed in the leaching such as hydrogen peroxide, for example. The washing can be followed with a rinse using de-ionized water, for example, and a subsequent drying.

After the first removal of residue such as magnesium, reactive intermediates, or other residue from the anode 24, an additional thermal treatment is conducted. In various embodiments, the thermal processing includes heating the anode in an oven or furnace at an elevated temperature of from about 1250 to 1550 degrees C. This provides a subsequent redistribution and removal of any remaining residue where a substantial fraction of any remaining residue migrates to the surface of the anode 24 for easy removal. The combined leaching and thermal processing optimize function of the anode 24 to 90% or more (including all subranges between 90% and 100%) of the forming voltage by eliminating residue which impacts the operation of the anode 24.

Applicants have found that prior treatments including a combination of deoxidation sintering and an acid leaching, such as those described in U.S. Patent No. 4,537,641, result in increased CV upon dielectric oxide formation. It was found that anodes produced using those described process steps exhibited significant gas evolution and high leakage currents upon charging in a suitable working electrolyte to voltages greater than about 80% of the formation voltage. Applicants then discovered that a separate vacuum thermal treatment step following the leaching process can result in significant reduction in gas evolution and leakage currents in finished anodes produced by subsequent dielectric oxide formation.

After the leaching and additional thermal treatment to redistribute and/or remove selected or all residual products on the anode 24, an anodic oxide is formed thereon by anodically polarizing the metal compact in a suitable electrolyte to produce a functional capacitor anode 24. This forms an amorphous, insulating oxide layer that serves as the capacitor dielectric. The applied forming voltage $V_f$ or potential during growth must increase to maintain a stable electric field as the oxide film thickness increases.

Once the oxide layer has been grown to a selected formation voltage, application of a potential significantly below $V_f$ results in negligible further oxide growth and current flow. Accordingly, $V_f$ sets an upper limit on the working voltage, $V_w$, of the finished capacitor as application of a higher voltage would result in further oxide growth rather than storage of charge. However, degradation mechanisms in the dielectric oxide tend to be accelerated by high electric field and $V_w$ is generally specified at a substantially lower value than $V_f$ to extend device lifetime in devices permanently charged at elevated temperatures. Larger $V_w / V_f$ ratios (such as 0.25 or greater) may be exploited in applications in which the device is held at a constant, comparatively low temperature and in which the device is at full charge for a comparatively short time, such as implantable medical devices.

The combined leaching and thermal treatment allows maximization of the $V_w / V_f$ ratio to 0.9 or greater. Such optimization has not yet been achieved with known techniques. Accordingly, the present teachings enhance function and manufacturability of the anode 24 and the capacitor 20. It is within the scope of the present teachings to include further processing, as is known in the art, such as a thermal treatment or annealing process, and a second anodic oxide formation step can be used with the anode 24.

The present teachings further provide methods of delivering a therapy to a patient. Referring to Figure 1, the various electrodes 10 and capacitors 20 formed according to the present teachings are useful in several space-critical applications. As a non-limiting example, an anode 24 can be incorporated into a capacitor 20 which can be connected to the implantable medical device system 100. A therapy can then be administered from the implantable medical device system 100 to the patient. The therapy can include exposing the capacitor to a voltage of from between 150 to 375 volts, and all sub-ranges in between. An exemplary therapy is treatment of an arrhythmia.

Example embodiments will now be described more fully with reference to the accompanying drawings.

EXAMPLES

Example 1:

An experimental lot of cylindrical test anodes with diameters of about 13 millimeters and a thickness of about 3 millimeters were dry-pressed using commercially available tantalum powder with a nominal specific charge of 30,000 microfaradvolts/gram. A tantalum lead wire was embedded during the pressing process. The anodes were de-oxidation sintered by exposure to magnesium vapor for nominally 6 hours at 1170 degrees C in a furnace configured specifically for this purpose. Upon cooling and removal from the de-oxidation/sintering furnace, the reaction products and any remaining magnesium on the anode surfaces were removed by leaching in a solution comprising 6 N sulfuric acid and about 3 volume percent hydrogen peroxide for 12 hours or more. The anodes were washed in a succession of de-ionized water baths and oven dried at 70 degrees C. The leached and dried anodes were then loaded into a conventional vacuum furnace with a tantalum hot zone and annealed at 1375 degrees C for one hour. Following the vacuum thermal treatment, the anodes were formed in a first formation process to a potential of 262 volts, washed in de-ionized water, heat treated for 1 hour in air at 450 degrees C, and reformed to 262 volts in a second formation process. The resistivity of the forming electrolyte was in the range of 1000 to 2000 ohm-cm. The formed anodes were given yet another series of de-ionized water washes and then again oven dried in air at 70 degrees C.

The anodes were then individually charged to a working potential of 255 V under constant current conditions at nominally 3 milliamps in a working electrolyte with a resistivity of about 35 ohm centimeter. After reaching 255 V, the anodes were held at potential for 5 minutes, discharged and held at 0 V for two minutes, and then recharged to 255 V and held for an additional 5 minutes. The leakage current was measured after the second 5 minute hold at working potential. Impedance spectroscopy was performed over a frequency range of from 0.1 to 1000 Hz in the same electrolyte for selected samples.

Comparative Example 2:

An experimental lot of test anodes was pressed using substantially identical powder and pressing parameters as the anodes described in Example 1. De-oxidation sintering, leaching, washing and drying were also identical to the processes used in Example 1. However, no 1375 degrees for 1 hour post de-oxidation sintering thermal treatment was performed prior to beginning the first anodic oxide formation process. All subsequent processing and testing steps were substantially identical to those used to prepare the Example 1 anodes.

Comparative Example 3:

Another experimental lot of test anodes was pressed, again using substantially identical powder and pressing parameters as the Example 1 anodes. Following pressing the anodes were placed in the conventional tantalum hot zone vacuum furnace and sintered at 1375 degrees C for 1 hour. Upon cooling the samples were de-oxidation sintered by exposure to magnesium vapor for 4 hours at 1170 degrees C under the same conditions used for the Example 1 samples. Subsequent leaching, drying, formation and testing processes were substantially identical to those used to prepare the Example 1 anodes.

Discussion of Example 1 and Comparative Examples 2 and 3:

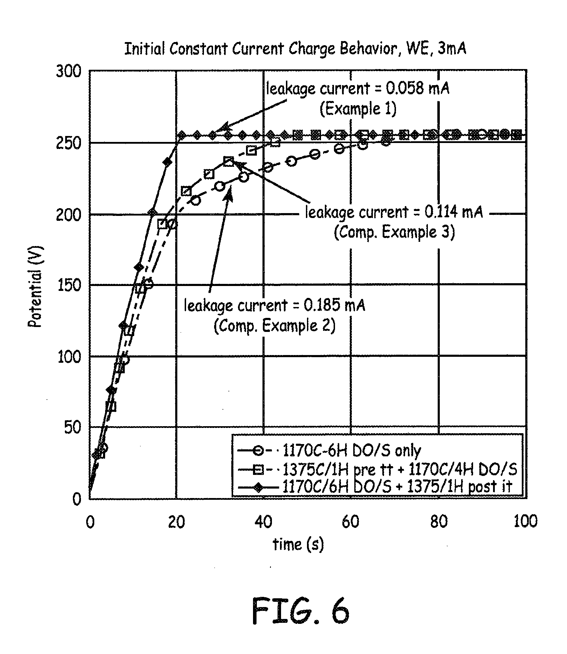

An ideal capacitor charged under constant current conditions should exhibit a linear increase in voltage with time up to the final voltage of interest. For the case of wet electrolytic capacitors, significant deviations from linearity at voltages less than $V_f$, the voltage used to form the anodic oxide, generally indicate the onset of undesirable electrochemical reactions. Figure 6 shows the initial voltage vs. time plots for a representative Example 1 anode as well as a representative anode of comparative Examples 2 and 3. The anode representative of the Example 1 processes exhibits substantially linear charging behavior with time. Both comparative Examples 2 and 3 exhibit significant deviations from linearity during charging, and considerable gas evolution is observed in the test cell as the anodes continue to draw the full 3 milliamp charging current for extended periods of time after the Example 1 anode is fully charged. The deviations from linearity for comparative Examples 2 and 3 becomes apparent at voltages greater than about 200 V, which corresponds to a $V_w/V_f$ ratio of about 0.75. In contrast, the Example 1 anode exhibits substantially linear behavior up to the test voltage of 255V, corresponding to a $V_w/V_f$ ratio of greater than 0.97. The samples of comparative Examples 2 and 3 also exhibit significantly higher leakage currents at the than the Example 1 sample, as is also depicted in Figure 6.

Example 4:

The data shown in Figure 6 were collected from three different de-oxidation sintering runs over several months. In order to rule out any differences among the deoxidation sintering runs, different powder lots used for pressing, etc., a group of test samples substantially identical to those described in Example 1 were pressed from the same powder lot with embedded lead wires and were de-oxidation/sintered in the presence of magnesium vapor at 1100 degrees C for 6 hours. The samples were acid leached, washed and dried as described in example one and separated into three groups. Two of the three groups were then given a vacuum thermal treatment at 1375 degrees C for 1 hour. Upon cooling, one of the groups which had received the vacuum thermal treatment was again placed in the de-oxidation/ sintering furnace and run through a second de-oxidation sintering process step at 1170 degrees C for 6 hours. These samples were again acid leached and dried. All three sample groups were then processed through a first formation step to 262 volts, washed, dried, heat treated and then processed through a second formation step to 262V as described in Example 1. The samples were then individually charged in - 35 ohm-cm working electrolyte under constant current conditions.

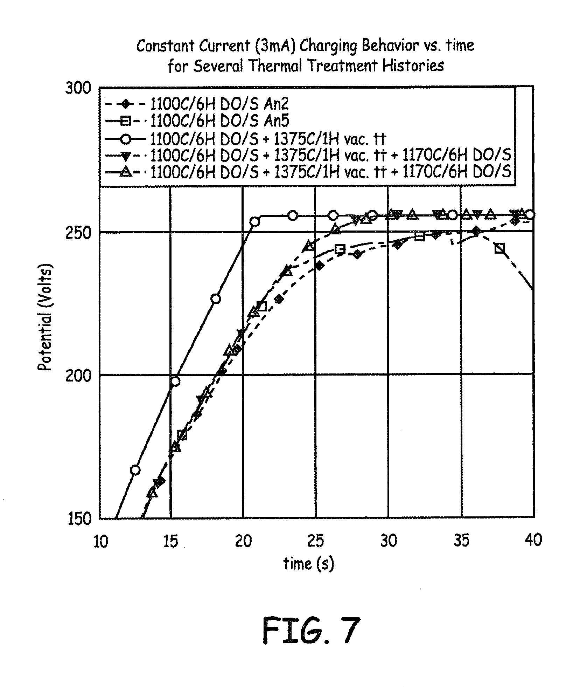

Charging data representative of the three sample groups are shown together in Figure 7. Consistent with the data of Figure 6, samples from the first group which experienced only a first de-oxidation sintering process exhibit strongly non-linear charging behavior, while samples from the second group which experienced a first de-oxidation sintering process and a subsequent vacuum thermal treatment exhibit nearly ideal charging behavior linearity. Significantly, samples from the third group which experience a second de-oxidation sintering process following vacuum thermal treatment again exhibit non-linear charging behavior that, while not as severe as that exhibited by the first sample group, is significantly degraded from that exhibited by the second sample group following vacuum thermal treatment. These data definitively demonstrate that the improved charging behavior observed for samples given a vacuum thermal treatment following deoxidation sintering is not simply due to sintering during the vacuum thermal treatment. The data in fact strongly suggest that a contaminant introduced during the de-oxidation sintering process is responsible for the observed charging non-linearity and that the vacuum thermal treatment serves to at least partially remove the impurity and/or mitigate its detrimental effects.

Example 5:

One impetus for pursuing de-oxidation sintering processes is the potential to increase volumetric energy density (VED) of capacitors utilized in space-critical applications. In order to demonstrate the benefits of the invented process, test anodes from two different split-lot de-oxidation sintering runs were compared to anodes processed by an optimized conventional sintering process. The sample preparation and thermal processing conditions employed are summarized in Table 1.

Table 1: Summary Process Description for Example 5 Samples

| Group ID | Process | Green Powder Weight (g) | Thermal Process 1 | Thermal Process 2 |
| --- | --- | --- | --- | --- |
| "CS" | Conventional vacuum sintering | 2.35, 2.45 | vacuum sinter | None |
| De-oxidation sintering Group 1 | De-oxidation sintering + vacuum thermal treatment | 2.25, 2.35 | 1170 C / 6 H de-oxidation sintering | 1375 C / 1 H vacuum thermal treatment |
| De-oxidation sintering Group 2 | de-oxidation sintering + vacuum thermal treatment | 2.35 | 1100 C / 6 H de-oxidation sintering | 1375 C / 15 min, 1275 C / 30 min vacuum thermal treatment |

The first formation, heat treatment, and second formation processes were substantially identical to those described in Example 1. All samples were pressed to the same nominal green volume. Delivered energy was determined in a working electrolyte with a nominal resistivity of 35 ohm-cm at 37 degrees C. The samples were wrapped in a porous polytetrafluoroethylene (PTFE) separator, sandwiched between two high-capacitance activated carbon cathodes, and secured with a spring clamp. Upon charging to 255V, the samples were discharged into a 100 ohm load resistor. The voltage and current traces were captured on a digital storage oscilloscope and the product of voltage and current were integrated over the discharge time to determine delivered energy.

The resulting data for representative samples from each group are as follows. The average increase in delivered energy for the two groups of de-oxidation sintering samples relative to the average delivered energy for the group of conventionally sintered samples is about 33%. This can result in a substantial volume decrease for high-voltage, high energy capacitors used in space critical applications such as high-power implantable medical devices. As the pressed powder volume is about 0.4 cm$^3$ for all samples, the delivered energy density relative to the as pressed powder volume averages about 10.5 J/cm$^3$ for the conventionally sintered anodes, while the delivered energy density for both groups of deoxidation sintering anodes averages about 14.0 J/cm$^3$.

Clarification Example 6:

Figures 6 and 7 demonstrate that the successive process steps of deoxidation/sintering, acid leaching to remove the de-oxidation sintering reaction product, and vacuum thermal treatment result in lower leakage currents and improved charging behavior at high $V_w/V_f$ ratios than the successive steps of vacuum thermal treatment, deoxidation/ sintering, and acid leaching or the successive steps of de-oxidation/sintering and acid leaching alone. As examined by scanning electron microscopy, the resulting microstructures for the three different process step sequences shown in Table 1 appear substantially identical. Based upon this observation it appears that substantially all sintering as defined herein occurs during the de-oxidation sintering process as the microstructure appears unchanged by an additional vacuum thermal treatment either before or after the de-oxidation sintering process. It is somewhat difficult to quantitatively compare non-uniform microstructures. However, as described by equation [2], capacitance is directly proportional to surface area. Consequently samples were prepared with the purpose of comparing the surface areas resulting from various process steps and combinations thereof. Specifically, test samples comprising 5.24 grams of the same powder used to prepare the Example 1 samples were pressed to a nominal green density of 5.70 grams/cm$^3$. The green powder compacts were then split into four equal sized groups and each group was exposed to a different thermal process step or steps, as described in Table 2:

Table 2: Thermal Process Steps for Comparative Example 6 Samples

| Group Number | First Process Step | Second Process Step |
| --- | --- | --- |
| 1 | 1170C / 5 hour de-oxidation sintering thermal treatment | None |
| 2 | 1170C / 5 hour vacuum thermal treatment | None |
| 3 | 1170C / 5 hour de-oxidation sintering thermal treatment | 1375C / 1 hour vacuum thermal treatment |
| 4 | 1375C / 1 hour vacuum thermal treatment | None |

All samples receiving a de-oxidation sintering thermal process step were acid leached, washed and dried as described in Example 1 prior to any further processing. Following completion of the various thermal processes, the groups were individually formed to a potential of 30 volts to form a uniform but relatively thin dielectric oxide while consuming a relatively small fraction of underlying tantalum metal. Following washing and drying, impedance spectra from 0.1 Hz to 1000 Hz were obtained in an electrolyte comprising 1N sulfuric acid.

It was found that in the frequency range below about 10 Hz where transport phenomena in the electrolyte are less pronounced, the capacitances (proportional to surface area) of the samples of groups 2 and 4 (subjected only to vacuum thermal treatments) are about 40-50% larger than those of the group 1 (de-oxidation sintering only) and group 3 (de-oxidation sintering + 1375 degrees C / 1 hour vacuum thermal treatment) for which processing included a de-oxidation/sintering step. Significantly, comparison of group 1 and group 3 reveals that the subsequent 1375 degrees C / 1 hour results in very little to substantially no further sintering (or surface area reduction) relative to that produced by de-oxidation sintering alone.

These data surprisingly show that the present teachings demonstrate significant differences and improvements over prior thought in which it is taught that a thermal treatment following a de-oxidation sintering process step provides additional sintering and microstructural refinement, such as the disclosure of U.S. Patent No. 6,447,570 and U.S. Patent Application Publication No. 2008/0145262 A1.

Clarification Example 7:

It was hypothesized in Example 4 that a contaminant introduced during the deoxidation sintering process which was subsequently reduced by the post de-oxidation sintering vacuum thermal treatment may be responsible for the observed improvements in electrical properties demonstrated for the post de-oxidation sintering vacuum thermal treatment. In order to test this hypothesis secondary ion mass spectrometry was employed to examine contamination levels in the near surface regions of 0.015 inch diameter tantalum lead wires immediately following de-oxidation sintering and leaching and then after the post de-oxidation sintering vacuum thermal treatment. Specifically, several wires were processed through de-oxidation/sintering at 1170 degrees C for 6 hours and were then acid-leached, washed and dried as described in Example 1. A subset of these wires was then subjected to vacuum thermal treatment at 1375 degrees C for 1 hour.

The experiments were performed on wires rather than an exemplary powdered metal compact as the secondary ion mass spectrometry (SIMS) analytical method employed requires a substantially planar surface with lateral dimensions of several hundred microns or more to function properly. A Cameca SIMS instrument (available from Cameca Instruments, Inc. of Nampa, Idaho) was used to perform SIMS depth profiling using oxygen primary ions. The detection limit of this instrument for magnesium under the analytical conditions employed is approximately $3 \times 10^{15}$ atoms/cm$^3$.

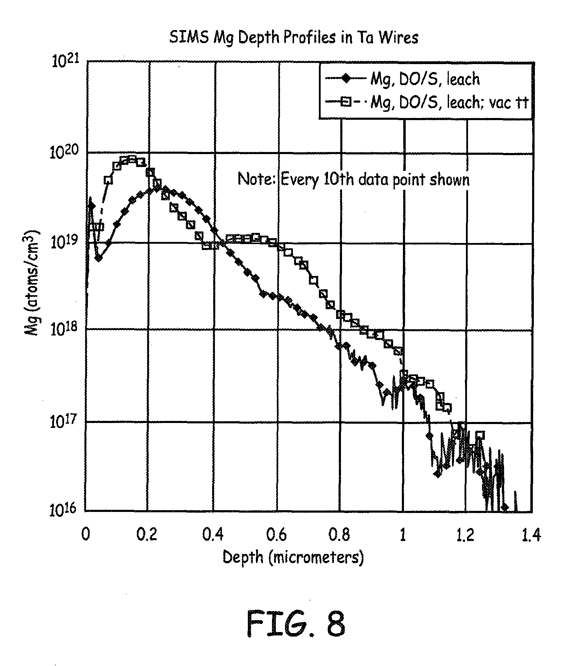

Figure 8 shows the experimental data that were obtained. The concentration quantification is based upon relative sensitivity factors obtained from known standards. Absolute quantification of SIMS data is difficult, particularly in the very near surface region at distances less than range of the primary beam in the solid (less than 20 nanometers for 8.5 KeV 02 in tantalum). The situation is further complicated by the presence of a native oxide which forms on the tantalum upon exposure to air, and it is also certain that any magnesium in the near surface region of the sample is also oxidized.

Nevertheless, Figure 8 clearly shows that a readily detectable quantity of magnesium has diffused into the near surface region of the tantalum wire during the deoxidation sintering process, when the magnesium activity at the surface is high due to the presence of magnesium vapor at a pressure of greater than 1 bar. It is also clear that the post de-oxidation sintering thermal treatment results in the redistribution of magnesium in the wire, with magnesium migrating both deeper into the solid and migrating back toward the surface since the magnesium activity (fugacity) in the vacuum furnace atmosphere is substantially zero.

Given that the temperatures employed in both the de-oxidation sintering process and the subsequent vacuum thermal treatment are of the order of 0.5 times the melting point of tantalum or less, it is highly likely that the majority of the magnesium migration occurs along crystalline defects such as grain boundaries. Additionally, although no phase diagram is available for the magnesium-tantalum binary system, it is generally acknowledged that the solubility of magnesium in the tantalum lattice must be very low, and solutes with low solubility are known to segregate to grain boundaries and free surfaces. It is believed that a significant fraction of the very near surface magnesium is oxidized, and magnesium oxide is known to be soluble in acidic solutions. The pH of the forming electrolyte employed in these studies is approximately 4, so it is highly likely that the near surface magnesium oxide dissolves when the anodes are exposed to the forming electrolyte both prior to and during the early stages of the formation process. This contention is supported by chemical analysis of the forming electrolyte in a formation tank used to produce anodes processed by de-oxidation/sintering and a substantially identical formation tank use to produce anodes processed by conventional vacuum sintering. The forming electrolyte in the tank used for processing de-oxidation sintering anodes was found to have more than an order of magnitude higher magnesium concentration than that of the tank used for producing conventionally sintered product.

As a consequence of the magnesium redistribution occurring during the post deoxidation sintering thermal treatment, less magnesium is available for incorporation into the amorphous oxide film formed during the anodization process. Reduced magnesium incorporation into the growing anodic film, particularly in regions near grain boundaries of the underlying metal may in large part be responsible for the improved charging behavior and lower leakage currents observed for anodes subjected to post de-oxidation sintering vacuum thermal treatment. It follows that acid leaching to remove as much surface and near surface magnesium and magnesium oxide as possible before rather than after vacuum thermal treatment may be advantageous.

Clarification Example 8:

We have found that a continuous protective magnesium oxide film is not formed in the invented process described herein. The sample was prepared substantially identically to the samples described in Example 1, but was removed from the process flow immediately following the de-oxidation sintering process, i.e., prior to acid leaching. The de-oxidation sintering tantalum metal microstructure is visible along with a number of small particles sitting on the tantalum metal surface. The particle surface coverage is less than 5% of the total surface area. Auger analysis which was localized to substantially only the particles reveals the presence of primarily magnesium, oxygen, and tantalum at distinct regions while other Auger analysis shows that substantially only tantalum and oxygen are detected. A magnesium signal is detected only when analyzing the particles. As Auger spectrometry is surface sensitive and substantially all magnesium is confined to the particles sitting on the metal surface, it is clear that no protective magnesium oxide film is formed during the process described herein.

As it has been demonstrated that no protective magnesium oxide film is formed in the invented process described herein, the removal of the magnesium oxide particles and any near-surface magnesium or magnesium reaction products before performing the vacuum thermal treatment step has no disadvantages and may be highly advantageous.

The invention is set out in the appended claims.

**Claims**

1.  A method of preparing an anode for use in a high volumetric energy density electrolytic capacitor, the method comprising:

    a. de-oxidizing and sintering a lead wire (32) in a valve metal powder compact to form the anode (24), where the de-oxidizing and sintering are conducted in the presence of a reactive metal having a stronger affinity for oxygen than the valve metal powder;
    b. removing residual reactive metal and at least one other contaminant from the anode surface with a leaching process;
    c. redistributing a remaining dissolved residual reactive metal residue in the anode by thermal processing after the leaching; and
    d. anodically forming a dielectric oxide on a surface of the anode, where the forming is conducted up to a specified forming voltage.

2.  The method of claim 1, wherein redistributing the residue within the anode comprises bringing at least some of the residue to a surface of the valve metal region.

3.  The method of claim 1, further comprising conducting the de-oxidizing and sintering in a de-oxidizing furnace.

4.  The method of claim 1, further comprising pre-treating a portion of the lead wire to improve bonding the lead wire to the valve metal.

5.  The method of claim 1, wherein the reactive metal comprises at least one of magnesium, calcium, and sodium.

6.  The method of claim 1, wherein the valve metal comprises tantalum.

7.  The method of claim 1, wherein the de-oxidation and sintering temperature ranges from between 1000 degrees C to 1300 degrees C.

8.  The method of claim 1, wherein the leaching further comprises treating the anode with an inorganic acid solution.

9.  A valve metal capacitor anode comprising:

    a valve metal lead wire (32) embedded within a metal powder compact, wherein the valve metal lead wire and metal powder compact are bonded together during a de-oxidation sintering process in the presence of a reactive metal vapor having a stronger affinity for oxygen than the metal powder and subsequently leached to remove a portion of the residue from the reactive metal vapor and thermally processed to redistribute remaining residue from the reactive metal vapor.

10. The valve metal capacitor anode of claim 9, wherein the lead wire has been pre-treated to de-oxidize the lead wire in the presence of a reactive metal vapor and to remove the reactive metal oxides formed prior to embedding the lead wire in the metal powder compact.

**Patentansprüche**

1.  Verfahren zum Herstellen einer Anode für die Verwendung in einem Elektrolytkondensator mit hoher volumetrischer Energiedichte, wobei das Verfahren umfasst:

a. Desoxidieren und Sintern eines Leitungsdrahtes (32) in einem Ventilmetallpulverpresskörper, um die Anode (24) zu bilden, wobei das Desoxidieren und das Sintern in Anwesenheit eines reaktiven Metalls mit einer stärkeren Sauerstoffaffinität als das Ventilmetallpulver ausgeführt wird;

b. Entfernen von restlichem reaktivem Metall und mindestens einer weiteren Verunreinigung von der Anodenoberfläche mit einem Auslaugungsverfahren;

c. Neuverteilen eines restlichen gelösten Rückstands des restlichen reaktiven Metalls in der Anode durch thermisches Aufbereiten nach dem Auslaugen; und

d. anodisches Bilden eines dielektrischen Oxids auf einer Oberfläche der Anode, wobei das Bilden bis zu einem bestimmten Bildungsvolumen ausgeführt wird.

2. Verfahren nach Anspruch 1, wobei das Neuverteilen des Rückstands innerhalb der Anode umfasst, zumindest einen Teil des Rückstands zu einer Oberfläche des Ventilmetallbereichs zu bringen.

3. Verfahren nach Anspruch 1, das ferner umfasst, das Desoxidieren und das Sintern in einem Desoxidationsofen auszuführen.

4. Verfahren nach Anspruch 1, das ferner eine Vorbehandlung eines Teils des Leitungsdrahtes umfasst, um das Verbinden des Leitungsdrahtes mit dem ventilmetall zu verbessern.

5. Verfahren nach Anspruch 1, wobei das reaktive Metall Magnesium und/oder Calcium und/oder Natrium enthält.

6. Verfahren nach Anspruch 1, wobei das Ventilmetall Tantal enthält.

7. Verfahren nach Anspruch 1, wobei die Desoxidations- und die Sintertemperatur in einem Bereich zwischen 1000 Grad C und 1300 Grad C liegen.

8. Verfahren nach Anspruch 1, wobei das Auslaugen ferner umfasst, die Anode mit einer anorganischen Säurelösung zu behandeln.

9. Ventilmetallkondensatoranode, mit:

einem Ventilmetallleitungsdraht (32), der innerhalb eines Metallpulverpresskörpers eingebettet ist, wobei der ventilmetallleitungsdraht und der Metallpulverpresskörper während eines Desoxidations-Sinter-Verfahrens in Anwesenheit eines reaktiven Metalldampfes mit einer stärkeren Sauerstoffaffinität als das Metallpulver miteinander verbunden werden und nachfolgend ausgelaugt werden, um einen Teil des Rückstands von dem reaktiven Metalldampf zu entfernen, und thermisch aufbereitet werden, um den restlichen Rückstand von dem reaktiven Metalldampf neu zu verteilen.

10. Ventilmetallkondensatoranode nach Anspruch 9, wobei der Leitungsdraht vorbehandelt wurde, um den Leitungsdraht in Anwesenheit eines reaktiven Metalldampfes zu desoxidieren und um das gebildete reaktive Metalloxid vor dem Einbetten des Leitungsdrahtes in den Metallpulverpresskörper zu entfernen.

**Revendications**

1. Procédé de préparation d'une anode pour une utilisation dans un condensateur électrolytique à densité d'énergie volumétrique élevée, le procédé comprenant :

a. de désoxyder et de fritter un fil conducteur (32) en un comprimé de poudre de métal valve pour former l'anode (24), la désoxydation et le frittage étant effectués en présence d'un métal réactif ayant une affinité plus forte pour l'oxygène que la poudre de métal valve ;

b. d'éliminer le métal réactif résiduel et au moins un autre contaminant de la surface de l'anode par un traitement de lixiviation ;

c. de redistribuer un résidu de métal réactif résiduel dissous restant dans l'anode par un traitement thermique après la lixiviation ; et

d. de former de manière anodique un oxyde diélectrique sur une surface de l'anode, où la formation est effectuée jusqu'à une tension de formation déterminée.

2. Procédé selon la revendication 1, dans lequel la redistribution du résidu à l'intérieur de l'anode comprend d'amener au moins une partie du résidu à une surface de la région de métal valve.

3. Procédé selon la revendication 1, comprenant en outre d'effectuer la désoxydation et le frittage dans un four de désoxydation.

4. Procédé selon la revendication 1, comprenant en outre de prétraiter une partie du fil conducteur afin d'améliorer la liaison du fil conducteur au métal valve.

5. Procédé selon la revendication 1, dans lequel le métal réactif comprend au moins l'un parmi du magnésium, du calcium et du sodium.

6. Procédé selon la revendication 1, dans lequel le métal valve comprend du tantale.

7. Procédé selon la revendication 1, dans lequel les plages de température pour la désoxydation et le frittage sont comprises entre 1000 degré Celsius et 1300 degrés Celsius.

8. Procédé selon la revendication 1, dans lequel la lixiviation comprend de traiter l'anode à l'aide d'une solution d'acide inorganique.

9. Anode de condensateur de métal valve comprenant :

un fil conducteur en métal valve (32) incorporé dans un comprimé de poudre de métal, dans lequel le fil conducteur en métal valve et le comprimé de poudre de métal sont liés ensemble au cours d'un processus de désoxydation et de frittage en présence d'une vapeur de métal réactif ayant une affinité plus forte pour l'oxygène que la poudre de métal et sont ensuite lixiviés pour éliminer une partie du résidu de la vapeur de métal réactif et traités thermiquement pour redistribuer les résidus restants à partir de la vapeur de métal réactif.

10. Anode de condensateur de métal valve selon la revendication 9, dans laquelle le fil conducteur a été traité pour désoxyder le fil conducteur en présence d'une vapeur de métal réactif et pour éliminer les oxydes de métal réactif formés avant l'incorporation du fil conducteur dans le comprimé de poudre de métal.

FIG. 1

FIG. 2

**FIG. 3**

FIG. 4

Schematic Comparison Specific Charge Decrease with
Formation Potential for "High" and "Low" CV powders

FIG. 5

FIG. 6

Constant Current (3mA) Charging Behavior vs. time
for Several Thermal Treatment Histories

FIG. 7

SIMS Mg Depth Profiles in Ta Wires

FIG. 8

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0245109 A
- US 3818581 A
- US 4537641 A
- US 42974909 A
- US 6447570 B
- US 20080145262 A1